Europäisches Patentamt

European Patent Office

Office européen des brevets

(11) Publication number: **0 256 813**
A1

# EUROPEAN PATENT APPLICATION

(21) Application number: 87307028.8

(22) Date of filing: 07.08.87

(51) Int. Cl.⁴: C 07 D 307/32

(30) Priority: 11.08.86 US 895116

(43) Date of publication of application:
24.02.88 Bulletin 88/08

(84) Designated Contracting States:
AT BE CH DE ES FR GB GR IT LI LU NL SE

(71) Applicant: DAVY McKEE (LONDON) LIMITED
250, Euston Road
London NW1 2PG (GB)

(72) Inventor: Keller, George Ernest, II
1207 Ellen Drive
South Charleston West Virginia 25303 (US)

Culp, Gary Lynn
Route 1, Box 25C
Kenna West Virginia 25248 (US)

Ho, Fun Gau
7 Ridge Road
Charleston West Virginia 25314 (US)

(74) Representative: Eyles, Christopher Thomas et al
BATCHELLOR, KIRK & EYLES 2 Pear Tree Court
Farringdon Road
London, EC1R 0DS (GB)

(54) Purification of gamma-butyrolactone.

(57) A process for separating dialkyl succinate from a mixture containing said dialkyl succinate and gamma-butyrolactone wherein the mixture is contacted with an organic extracting agent to selectively remove dialkyl succinate from the feed mixture and produce a gamma-butyrolactone enriched raffinate. The extract and raffinate phases thereafter are distilled to recover extraction solvents and produce a dialkyl succinate stream for recycle and a product gamma-butyrolactone stream.

Fig.1.

EP 0 256 813 A1

**Description**

## PURIFICATION OF GAMMA-BUTYROLACTONE

BACKGROUND OF THE INVENTION

### 1. Field of the Invention
This invention relates to an energy efficient method of recovering gamma-butyrolactone (GBL) from mixtures containing gamma-butyrolactone and dialkyl esters of succinic acid. More particularly, this invention relates to a method of recovering gamma-butyrolactone by liquid-liquid extraction from a mixture of gamma-butyrolactone and dialkyl esters of succinic acid obtained, for example, by catalytically hydrogenating a dialkyl maleate.

### 2. Description of Related Art
1,4-butanediol, a commercially valuable monomer for preparing polyesters, can be prepared by catalytically hydrogenating dialkyl maleates obtained by esterifying maleic acid or maleic anhydride with lower alkanols. In addition to the desired 1,4-butanediol, the crude hydrogenation product also contains, inter alia, tetrahydrofuran (THF), and gamma-butyrolactone (GBL) as commercially useful by-products.

Recovery of gamma-butyrolactone in high purity, i.e., greater than 90 wt.% and preferably greater than 95 wt.%, from the hydrogenation product stream, however, generally is complicated by the presence of dialkyl succinates, another common hydrogenation product, with which gamma-butyrolactone forms an azeotrope. The alkyl moiety of the dialkyl succinate typically corresponds to the alkanol used initially to esterify the maleic acid or maleic anhydride. While separation of gamma-butyrolactone from dialkyl succinates might be accomplished using known azeotropic distillation techniques, the capital cost and energy demands for such processes are high. Thus, it would be beneficial if a less energy intensive process was available to perform this separation. The present invention is based on the discovery that a combination of liquid-liquid extraction and distillation can be used to accomplish this separation more economically than can azeoptropic distillation.

### BRIEF DESCRIPTION OF THE DRAWINGS
Figure 1 illustrates a schematic flow diagram of one embodiment of the present invention wherein a feed mixture containing dialkyl succinate and gamma-butyrolactone is contacted in an extractor with an organic extracting agent.

Figure 2 illustrates a schematic flow diagram of a preferred embodiment of the present invention wherein the feed mixture is treated in a dual solvent extraction process using both a polar solvent, such as water, and an organic extracting agent.

Figure 3 schematically illustrates an alternative arrangement for carrying out the dual solvent extraction.

### DESCRIPTION OF THE INVENTION
In accordance with the present invention, a process for separating dialkyl succinate from a mixture containing said dialkyl succinate and gamma-butyrolactone is provided which comprises intimately contacting said mixture with an organic extracting agent which is substantially immiscible with said gamma-butyrolactone to extract dialkyl succinate from said mixture; allowing the organic extracting agent containing extracted dialkyl succinate to separate as an extract phase from dialkyl succinate-depleted, contacted mixture; separately recovering said extract phase and the dialkyl succinate-depleted, contacted mixture as a raffinate phase; distilling said extract phase to separate dialkyl succinate from said organic extracting agent and recycling said organic extracting agent for contacting said mixture, and distilling said raffinate phase to recover a gamma-butyrolactone product.

In a preferred aspect of the present invention, the process for separating dialkyl succinate from the mixture of dialkyl succinate and gamma-butyrolactone further includes the step of contacting the organic extracting agent containing removed dialkyl succinate as an extract phase with a polar solvent, such as water, to extract gamma-butyrolactone from the extract phase and thus improve the recovery of gamma-butyrolactone. Preferably, the water containing extracted gamma-butyrolactone then is combined with the mixture of dialkyl succinate and gamma-butyrolactone for further contacting with said organic extracting agent. The presence of the aqueous (polar) phase improves the capacity of the organic extracting agent for dialkyl succinates.

The present invention relates to a process for recovering gamma-butyrolactone (GBL) from mixtures containing gamma-butyrolactone and dialkyl esters of succinic acid. Such mixtures can be produced by conventional distillation techniques from a crude 1,4-butanediol product obtained by catalytically hydrogenating dialkyl maleates. For example, hydrogenation of diethyl maleate yields a crude product which includes 1,4-butanediol, diethyl succinate, gamma-butyrolactone, tetrahydrofuran, ethanol, butanol, and water. Employing routine distillation procedures, a mixture containing primarily gamma-butyrolactone and diethyl succinate can be recovered from the crude 1,4-butanediol product. If any 1,4-butanediol remains in the mixture, it will be recovered with gamma-butyrolactone in the process of this invention and readily can be removed subsequently from the gamma-butyrolactone product using standard distillation procedures.

The present invention specifically is directed to a liquid-liquid extraction process for recovering gamma-butyrolactone from mixtures containing gamma-butyrolactone and dialkyl succinates. The method is particularly applicable to mixtures containing dialkyl succinates having from about 1 to about 6 carbons in the alkyl moiety. For ease in presentation, the invention will be described with reference to a mixture containing gamma-butyrolactone and diethyl succinate, although as will be recognized by those skilled in the art, the procedures and techniques disclosed herein are applicable to the recovery of gamma-butyrolactone from mixtures containing other dialkyl succinates.

In accordance with the present invention, the feed mixture of diethyl succinate and gamma-butyrolactone is contacted intimately with an organic extracting agent which is substantially immiscible with gamma-butyrolactone. Organic extracting agents which may be employed in practicing the process of the present invention include aliphatic, and cycloaliphatic (i.e., alicyclic) hydrocarbons having up to 20 carbon atoms and preferably having from about 6 to about 12 carbon atoms. Particularly useful organic extracting agents can be selected from the alkanes and cycloalkanes. Specific examples of organic extracting agents suitable for practicing the present invention are hexane, heptane, octane, decane, cyclohexane and various mixtures thereof.

Preferably, an organic extracting agent is selected which is liquid at the ordinary operating conditions of the extraction, which are described hereafter, and has a relatively low boiling point. The low boiling point facilitates subsequent distillation of the extract phase using a minimum quality of energy and with a minimum degradation of any temperature-sensitive constituents. Generally, an organic extracting agent having a boiling point of no lower than about 60 to 65° C is preferred so that ordinary cooling water can be utilized as the condensing medium at the operating pressure of the distillation. Also, based on energy considerations, the boiling point of the organic extracting agent should not be higher than the boiling point of the dialkyl succinate in the feed mixture. Typically, an organic extracting agent having a boiling point between about 80° and about 180°C is preferred.

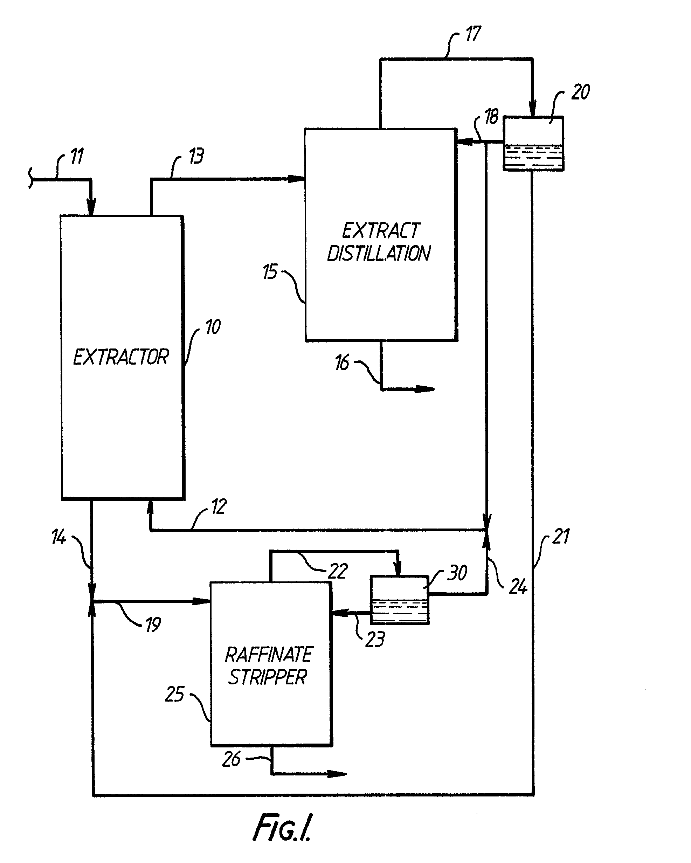

The diethyl succinate and gamma-butyrolactone feed mixture is contacted intimately in an extraction zone with the organic extracting agent to extract diethyl succinate into the organic extract phase. Thereafter, the two phases are allowed to separate. The extraction can be accomplished using a wide variety of known techniques and conventional equipment. Preferably, the extraction is carried out in a continuous manner using either continuous countercurrent multi-stage contacting procedures employing a series of mixer-settlers or a trayed column or by countercurrent differential contact in a packed tower, rotary-disc contacting column and the like. Equipment suitable for accomplishing the extraction will be apparent to those skilled in the art and need not be described in any detail. Arrangements for conducting a continuous extraction are illustrated schematically in Figures 1, 2 and 3.

Referring first to Figure 1, the feed mixture containing diethyl succinate and gamma-butyrolactone is introduced through line 11 into the top of extraction zone 10 wherein it is contacted intimately with an organic extracting agent such as heptane in order to remove diethyl succinate from the feed mixture. The heptane is introduced through line 12 at the bottom of the extraction zone. Typically, the feed mixture will contain anywhere from about 0.5 weight percent up to about 50 weight percent diethyl succinate. Normally, the feed mixture will contain from about 5 to about 40 weight percent diethyl succinate.

Flow of the two streams through extraction zone 10 of Figure 1 is countercurrent. While countercurrent extraction techniques are preferred, other known extraction techniques also could be employed and modifications thereof needed to obtain a desired degree of separation will be apparent to those skilled in the art. An extract phase containing the organic extracting agent and the dialkyl succinate extracted from the feed mixture is recovered from extraction zone 10 in line 13 and a raffinate phase containing gamma-butyrolactone is recovered from extraction zone 10 through line 14.

The weight ratio of the organic extracting agent introduced into extracting zone 10 to the feed mixture will vary somewhat depending upon the amount of diethyl succinate contained in the feed mixture and the desired degree of its removal. Generally, a weight ratio of organic extracting agent to feed mixture of from about 3:1 to about 20:1 generally is satisfactory in the Figure 1 embodiment, with a weight ratio of about 4:5:1 to about 13:1 being preferred. Because of the high degree of separation of diethyl succinate from gamma-butyrolactone obtained in the process of this invention, as few as 2 and up to about 25 theoretical equilibrium stages are used in extraction zone 10 depending on the level of diethyl succinate in the feed mixture and the degree of separation desired. Generally, from about 5 to about 15 theoretical equilibrium stages are used. In order to maintain a specified separation, higher solvent to feed weight ratios are required as the number of theoretical equilibrium stages is lowered. However, with fewer equilibrium stages, the recovery of gamma-butyrolactone tends to decrease.

The temperature for extracting diethyl succinate from the feed mixture in extraction zone 10 is not critical. The best temperature will depend primarily upon the choice of the organic extracting agent (e.g. conditions under which it is liquid) and the nature and quantity of the dialkyl succinate being extracted. For example, when removing diethyl succinate from gamma-butyrolactone, a temperature in extraction zone 10 of from about ambient up to about 100°C is satisfactory, with a temperature between about ambient and 60°C being preferred. Generally, the extraction zone is operated at about atmospheric pressure although any pressure at which the two liquid streams are maintained in the liquid state can be employed.

The organic extract phase recovered in line 13 from extraction zone 10 is distilled in extract distillation zone 15 to recover the organic extracting agent for reuse in the extraction process separate from a substantially solvent-free extract containing diethyl succinate. In this embodiment, the extract phase typically will have anywhere from about 0.1 to 15 percent by weight diethyl succinate, and normally from about 0.5 to 10 percent by weight, with the balance being primarily the organic extracting agent. The solvent-free extract is recovered from the bottom of distillation zone 15 in line 16. Typically, the solvent-free extract stream is recycled to the hydrogenation reactor of the process for producing 1,4-butanediol.

The overhead in line 17 from distillation zone 15, after passing through conventional condensation equipment (not shown) is decanted in separator 20. One portion of the light fraction recovered from separator 20, comprising the organic extracting agent, is returned to distillation zone 15 as reflux through conduit 18, while the remaining portion is recirculated through conduit 12 to extraction zone 10. Additional organic extracting agent (not shown) also will be added to the recycle stream, as needed, to offset any losses thereof in the diethyl succinate and gamma-butyrolactone products. The heavy fraction recovered from separator 20 in line 21 contains gamma-butyrolactone and forms part of the feed to raffinate distillation zone 25.

The operating conditions for extract distillation zone 15 depend primarily on the particular solvent used for extraction. Generally, the distillation will be conducted at atmospheric pressure or lower. Depending upon the temperature sensitivity (i.e., chemical stability) of materials present in the extract phase, subatmospheric pressures may be preferred.

The raffinate phase from the bottom of extraction zone 10 is delivered through line 19 to raffinate distillation zone 25. Generally, in this embodiment the feed to distillation zone 25 will contain anywhere from about 0.05 up to about 10 weight percent diethyl succinate and more typically will contain from about 0.1 to 5 weight percent, with the balance being primarily gamma-butyrolactone. In this distillation zone, the organic extracting agent is recovered as an overhead product in line 22 and a gamma-butyrolactone product is recovered from the raffinate phase as the bottoms product in conduit 26.

The overhead from distillation zone 25 in conduit 22, after passing through conventional condensation equipment (not shown) is decanted in separator 30. The heavy fraction containing gamma-butyrolactone, is returned as reflux through line 23 to distillation zone 25 while the light fraction containing the organic extracting agent is recycled through line 24 to extraction zone 10.

The raffinate distillation generally will be conducted under similar conditions used in the extract distillation. The primary purpose of this distillation is to strip residual organic extracting agent from the gamma-butyrolactone enriched raffinate recovered from the base of extraction zone 10. The gamma-butyrolactone product recovered from distillation zone 25 typically will have the same level of diethyl succinate as the bottoms product recovered from extraction zone 10.

As those skilled in the art recognize, operating temperatures in the extract and raffinate distillation zones will be dependent upon the pressures used and both will be somewhat dependent upon the composition of the extract and raffinate phases being treated. Heat energy needed to effect the separations in distillation zones 15 and 25 can be supplied using one of a variety of reboiler options wellknown to those skilled in the art. Also as briefly mentioned above, if the feed to either the extract distillation zone or the raffinate distillation zone contains temperature sensitive materials, it is preferred to operate the distillations at a subatmospheric pressure, i.e., under a vaccuum. For example, a suitable operating pressure in such circumstances for the distillation zones 15 or 25 may be approximately 10-400 millimeters of mercury. Also, conventional equipment such as packed columns and sieve trays can be used for both the extract and raffinate distillation zones.

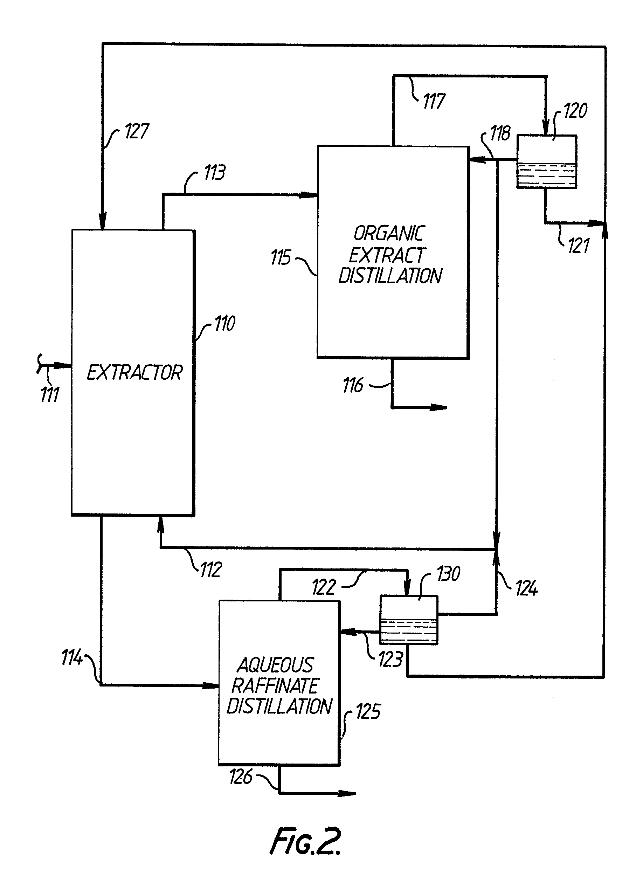

Figure 2 illustrates a preferred arrangement of the present invention referred to as the dual-solvent process. Components in Figure 2 similar to or identical with elements in the Figure 1 embodiment are identified using the same reference numeral increased by 100. In this embodiment to maximize recovery of gamma-butyrolactone, the organic extracting agent containing the extracted diethyl succinate is back-extracted with a polar solvent, such as water or another like-acting polar solvent such as acetonitrile which is substantially immiscible with the organic extracting agent and has an affinity for gamma-butyrolactone. The process is conducted in substantially the same manner as illustrated in Figure 1 using similar equipment and conditions except that the feed mixture of diethyl succinate and gamma-butyrolactone is fed through line 111 to an intermediate point of extraction zone 110. As recognized by those skilled in the art, the optimum placement of the feed depends on several variables and the location can be determined readily by those skilled in the art. In this embodiment, extraction zone 110 typically will have approximately 1 to 6 and normally 2 to 5 theoretical equilibrium stages above the feed point, while there may be anywhere from about 4 to 20 and ordinarily from about 6 to 15 theoretical equilibrium stages below the feed (including the feed stage itself).

In this embodiment, the organic extracting agent, such as heptane or decane, is introduced into the bottom of extraction zone 110 through line 112, while the polar solvent, e.g. water, is introduced into the top of extraction zone 110 through line 127. The aqueous (polar) stream passes downwardly through extraction zone 110 becoming enriched with gamma-butyrolactone, while the organic extracting agent flows countercurrently thereto and becomes enriched with diethyl succinate. Applicants have found that in addition to enhancing the recovery of gamma-butyrolactone from the organic extracting agent in the upper portion of extraction zone 110, water also increases the distribution coefficient of diethyl succinate in the lower portion of extraction zone 110 so that the quantity of organic extracting agent is reduced.

In this embodiment, the weight ratio of organic extracting agent introduced into extraction zone 110 to feed mixture generally will be between about 0.3:1 to about 15:1 although both higher and lower ratios can be used, and more preferably will be between about 0.5:1 and about 10:1. The weight ratio of water introduced into extraction zone 110 to feed mixture generally will be between about 0.15:1 to about 2:1 and preferably will be between about 0.2:1 and 1:1.

The organic extracting agent containing extracted diethyl succinate recovered in conduit 113 is distilled in extract distillation zone 115. After passing through a conventional condenser (not shown) the overhead fraction in line 117 is decanted into a light organic fraction and a heavy aqueous fraction in separator 120. As in the Figure 1 embodiment, diethyl succinate is recovered in line 116 as a bottoms product and is recycled to the dialkyl maleate hydrogenation reactor, while the organic extracting agent recovered as the light fraction from separator 120 is divided between reflux for distillation zone 115 through line 118 and recycle for extraction zone 110 through line 112. The aqueous stream recovered as the heavy fraction is recirculated through conduit 121 to extraction zone 110 as a portion of the water feed.

The bottoms product from extraction zone 110 recovered in line 114 comprises an aqueous raffinate phase containing gamma-butyrolactone. This stream is distilled in the aqueous raffinate distillation zone 125 to yield a bottoms gamma-butyrolactone product in line 126 and an overhead stream in line 122 which, after passing through a conventional condenser (not shown), is decanted in separator 130 to recover a heavy fraction comprising water, one portion of which is recycled through line 127 to extraction zone 110 and another portion of which is returned through line 123 as reflux for distillation zone 125, and a light fraction comprising organic extracting agent which is recycled through line 124 to extraction zone 110.

The conditions of operation of extraction zone 110 and distillation zones 115 and 125 and the equipment used are substantially the same as those described in connection with the Figure 1 embodiment. As was the case with Figure 1, the distillation zones also contain reboiler equipment needed to supply heat energy to distillation zones 115 and 125.

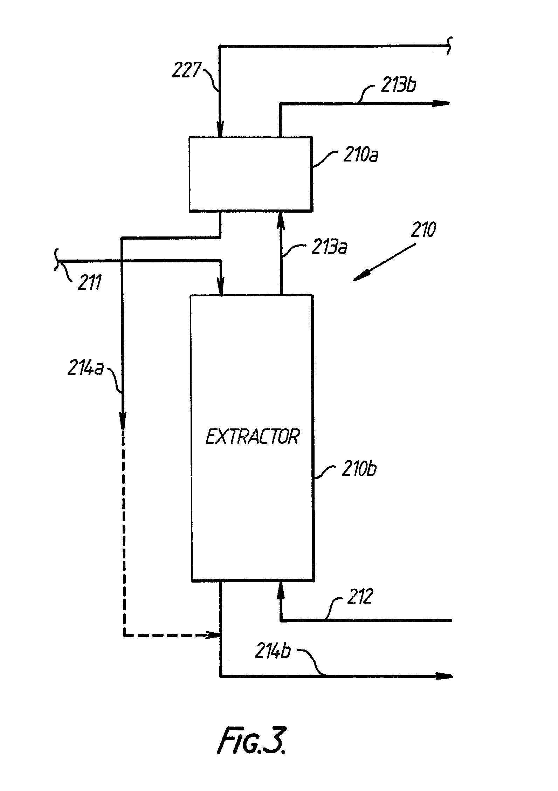

Figure 3 illustrates an alternate arrangement for the extraction zone 110 of the dual solvent process. As shown, extraction zone 210 is physically decoupled into sub-zones 210a and 210b. Diethyl succinate and gamma-butyrolactone feed mixture is fed to the top of sub-zone 210b through line 211 and is intimately contacted therein with organic extracting agent. The organic extracting agent is introduced through line 212 into the bottom of sub-zone 210b for upward flow therethrough countercurrent to the feed mixture. As in the Figure 2 arrangement, gamma-butyrolactone enriched raffinate is recovered at the bottom of extraction zone 210b in line 214b and is treated thereafter in distillation equipment (not shown) to remove organic extracting agent, which is recycled, from the gamma-butyrolactone product.

In the Figure 3 arrangement, an organic extract phase is recovered in line 213a. The organic extract phase is enriched in diethyl succinate and contains a minor amount of gamma-butyrolactone. This extract phase is contacted with water or a similarly acting polar solvent in extraction sub-zone 210a. The water is introduced through line 227 and strips gamma-butyrolactone from the organic extracting agent thus improving the overall recovery of gamma-butyrolactone. An aqueous raffinate phase containing gamma-butyrolactone. An aqueous raffinate phase containing gamma-butyrolactone is recovered in line 214a and either can be combined with the raffinate recovered in line 214b or separately treated to recover a gamma-butyrolactone product from the water. The organic extracting agent containing extracted dialkyl succinate is recovered in conduit 213b and is treated in distillation equipment (not shown) to recover separately organic extracting agent and diethyl succinate.

The present invention makes it possible to recover gamma-butyrolactone in high purity, i.e., greater than 90 wt. % and preferably greater than 95 wt. % from a mixture containing gamma-butyrolactone and up to about 50 wt. % dialkyl succinate.

The following Examples are intended to illustrate the present invention without limiting its scope in any way, which is defined in the appended claims.

EXAMPLE 1

In this Example, well-known, batch countercurrent multistage extraction procedures were used to simulate an eight equilibrium stage continuous liquid-liquid extraction process operated in accordance with the Figure 2 embodiment. For details please refer to Treybal, R.E., Liquid Extraction, 2nd Ed. Ch. 9 (1963). The feed mixture contained 62.8 weight percent gamma-butyrolactone and 37.2 by weight diethyl succinate and was contacted in accordance with such known procedures with heptane and water using an organic extracting agent to feed mixture weight ratio of 1.5 and a water to feed mixture weight ratio of 0.4. The procedure was designed to simulate the introduction of feed mixture on stage 6 of the process. The various streams of each batch stage were contacted in a separatory funnel for 30 minutes to insure that equilibrium conditions had been reached. The composition in percent by weight of the extract phase and raffinate phase which were recovered from equilibrium stages 8 and 1 respectively, of the batch simulation are presented in the following Table 1.

## TABLE 1

|  | Extract Phase | Raffinate Phase |
|---|---|---|
| Heptane | 90.26 | 0.07 |
| Diethyl succinate | 9.66 | 4.60 |
| Gamma-butyrolactone | 0.03 | 66.46 |
| Water | 0.05 | 28.87 |

In carrying out this extraction, it was observed that three immiscible phases formed on what would correspond to the feed tray (equilibrium stage 6) as well as on equilibrium stage 7 (first stage above the feed). Conditions preferably are selected to avoid formation of these three immiscible phases.

EXAMPLE 2

The procedure of Example 1 was repeated except that the weight ratio of heptane organic extracting agent to feed mixture was increased from 1.5 to 3.0. Otherwise, the operation was unchanged. The compositions in percent by weight of the extract and raffinate phases recovered from equilibrium stages 8 and 1 respectively of the batch simulation extraction zone are presented in the following Table 2.

## TABLE 2

|  | Extract Phase | Raffinate Phase |
|---|---|---|
| Heptane | 89.72 | 0.02 |
| Diethyl succinate | 10.20 | 0.53 |
| Gamma-butyrolactone | 0.08 | 71.39 |
| Water | < 0.01 | 28.06 |

In carrying out the method of this Example, two phases were observed on each equilibrium stage. The third phase observed on equilibrium stages 6 and 7 in the Example 1 process did not appear.

EXAMPLE 3

This example presents tabulated results of a computer simulation of the extraction process of the embodiment of Figure 1. Tables 3 and 4 summarize the number of theoretical equilibrium stages and the organic extracting agent to feed mixture weight ratios suitable for producing a raffinate stream having more than 95 wt. % gamma-butyrolactone and about 0.1 wt. % of diethyl succinate from feed mixtures containing respectively 60 wt. % gamma-butyrolactone and 40 wt. % of diethyl succinate and 95 wt. % gamma-butyrolactone and 5 wt. % of diethyl succinate. The percent recovery of gamma-butyrolactone in the raffinate recovered from the extractor also is reported.

## TABLE 3

### 40 wt. % Diethyl Succinate Feed

| No. Theoretical Equilibrium Stages | Solvent: Feed Weight Ratio | Gamma-butyrolactone percent recovery |
|---|---|---|
| 20 | 4.6 | 86.1 |
| 15 | 5.1 | 85.3 |
| 10 | 6.3 | 83.0 |
| 8 | 7.4 | 81.0 |
| 6 | 9.9 | 76.4 |
| 5 | 12.6 | 71.5 |

## TABLE 4

### 5 wt. % Diethyl Succinate Feed

| No. Theoretical Equilibrium Stages | Solvent: Feed Weight Ratio | Gamma-butyrolactone percent recovery |
|---|---|---|
| 20 | 6.1 | 92.5 |
| 15 | 6.5 | 92.1 |
| 12 | 6.9 | 91.5 |
| 10 | 7.4 | 91.0 |
| 8 | 8.2 | 90.0 |
| 4 | 14.3 | 83.0 |

Example 4

This example presents tabulated results of a computer simulation of the dual solvent extraction process of the Figure 2 embodiment. Tables 5 and 6 summarize the number of theoretical equilibrium stages, the organic extracting agent to feed mixture weight ratios and the water to feed mixture weight ratios suitable for producing a raffinate stream having about 0.1 wt. % diethyl succinate and an extract stream having about 0.1 wt. % gamma-butyrolactone from feed mixtures containing respectively 60 wt. % gamma-butyrolactone and 40 wt. % diethyl succinate and 95 wt. % gamma-butyrolactone and 5 wt. % diethyl succinate. The percent recovery of gamma-butyrolactone in the raffinate recovered from the extractor was above 99 wt. % in all cases.

## TABLE 5

### 40 wt. % Diethyl Succinate Feed

| No. Theoretical Equilibrium Stages Above Feed | Below Feed[1] | Organic Solvent: Feed Weight Ratio | Water: Feed Weight Ratio |
|---|---|---|---|
| 4 | 20 | 1.7 | 0.25 |
| 3 | 20 | 1.0 | 0.5 |
| 2 | 20 | 0.6 | 1.0 |
| 3 | 15 | 1.1 | 0.5 |
| 4 | 10 | 2.5 | 0.25 |
| 2 | 10 | 0.9 | 1.0 |
| 4 | 6 | 4.1 | 0.25 |
| 4 | 5 | 5.4 | 0.25 |
| 3 | 5 | 2.9 | 0.5 |
| 2 | 5 | 1.9 | 1.0 |

## TABLE 6

### 5 wt. % Diethyl Succinate Feed

| No. Theoretical Equilibrium Stages Above Feed | Below Feed[2] | Organic Solvent: Feed Weight Ratio | Water: Feed Weight Ratio |
|---|---|---|---|
| 6 | 20 | 3.2 | 0.25 |
| 4 | 20 | 1.2 | 1.0 |
| 6 | 10 | 3.8 | 0.25 |
| 4 | 10 | 1.5 | 1.0 |
| 6 | 5 | 6.0 | 0.25 |
| 4 | 4 | 2.8 | 1.0 |

---

[1]    Includes Feed Stage.

[2]    Includes Feed Stage.

While certain specific embodiments of the invention have been described with particularly herein, it will be recognized that various modifications thereof will occur to those skilled in the art. Therefore, the scope of the

0 256 813

invention is to be limited solely by the scope of the appended claims.

## Claims

1. A process for separating dialkyl succinate from a mixture containing said dialkyl succinate and gamma-butyrolactone comprising:

(a) intimately contacting said mixture with an organic extracting agent, which is substantially immiscible with said gamma-butyrolactone, to extract dialkyl succinate from said mixture;

(b) allowing the organic extracting agent containing extracted dialkyl succinate to separate as an extract phase from dialkyl succinate-depleted, contacted mixture;

(c) separately recovering said extract phase and the dialkyl succinate-depleted, contacted mixture as a raffinate phase;

(d) distilling said extract phase to separate dialkyl succinate from said organic extracting agent; and

(e) distilling said raffinate phase to recover gamma-butyrolactone product.

2. A process according to claim 1, which further includes the step of contacting the organic extracting agent containing extracted dialkyl succinate with a polar solvent to extract gamma-butyrolactone from said organic extracting agent.

3. The process of claim 2 wherein said polar solvent is water.

4. The process of claim 3 wherein the water containing extracted gamma-butyrolactone is combined with said mixture containing dialkyl succinate and gamma-butyrolactone.

5. The process of claim 3 wherein the water containing extracted gamma-butyrolactone is combined with the raffinate phase recovered in step (c).

6. The process of claim 4 or claim 5 wherein the distilling of said raffinate phase to recover a gamma-butyrolactone product also produces a water product.

7. The process of claim 6 wherein said water is used for contacting said organic extracting agent containing extracted dialkyl succinate.

8. The process of any one of claims 1 to 7, wherein said organic extracting agent is selected from the group consisting of aliphatic hydrocarbons, cycloaliphatic hydrocarbons, and mixtures thereof.

9. The process of claim 8 wherein said organic extracting agent has from 6 to 12 carbon atoms.

10. The process of claim 8 or claim 9 wherein said organic extracting agent is selected from the group consisting of alkanes, cycloalkanes, and mixtures thereof.

11. The process of any one of claims 1 to 10 wherein said distilling of step (d) is done at a sub-atmospheric pressure.

12. The process of any one of claims 1 to 11 wherein said distilling of step (e) is done at a sub-atmospheric pressure.

13. The process of any one of claims 1 to 12 wherein said organic extracting agent separated by distillation in step (d) is used to contact said mixture in step (a).

14. The process of any one of claims 1 to 13 wherein said dialkyl succinate is selected from the group consisting of diethyl, dipropyl and dibutyl succinate.

15. A process for separating dialkyl succinate from a mixture containing said dialkyl succinate and gamma-butyrolactone comprising,

(a) providing an extraction zone for contacting two immiscible liquids, said extraction zone having a first end and a second end;

(b) feeding said mixture to an intermediate point in said extraction zone between said first and second ends;

(c) intimately contacting said mixture in said extraction zone with an organic extracting agent, which is substantially immiscible with said gamma-butyrolactone, to extract dialkyl succinate from said mixture, said organic extracting agent having been fed to the first end of said extraction zone for flow therethrough,

(d) feeding a polar solvent to the second end of said extraction zone for intimately contacting said organic extracting agent and for flow through said extraction zone in a direction counter current to the flow of said organic extracting agent,

(e) separately recovering from said extraction zone a raffinate phase comprising a mixture of dialkyl succinate-depleted contacted mixture and polar solvent and an extract phase comprising organic extracting agent containing extracted dialkyl succinate,

(f) distilling said extract phase to separate dialkyl succinate from said organic extracting agent; and

(g) distilling said raffinate phase to recover a gamma-butyrolactone product.

16. The process of claim 15 wherein said polar solvent is water.

9

*Fig.I.*

EXTRACTOR — 10
11
13
15 EXTRACT DISTILLATION
16
17
18
20
12
14
19
22
30
23
24
21
RAFFINATE STRIPPER — 25
26

*Fig.2.*

0256813

Fig.3.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X,Y | US-A-2 875 213 (G.B. ULVILD) <br> * Whole document * | 1-16 | C 07 D 307/32 |
| Y | US-A-4 032 458 (S.D. COOLEY) <br> * Whole document; particularly column 8, lines 53-68; columns 9,10 * | 1-16 | |
| | | | **TECHNICAL FIELDS SEARCHED (Int. Cl.4)** |
| | | | C 07 D 307/00 |

The present search report has been drawn up for all claims

| Place of search | Date of completion of the search | Examiner |
|---|---|---|
| THE HAGUE | 03-11-1987 | ALLARD M.S. |